(19) Europäisches Patentamt
European Patent Office
Office européen des brevets

(11) EP 2 213 368 B1

# (12) EUROPEAN PATENT SPECIFICATION

(45) Date of publication and mention of the grant of the patent:
**01.05.2013 Bulletin 2013/18**

(51) Int Cl.:
***B01J 19/14*** *(2006.01)* ***B65D 81/20*** *(2006.01)*
***C07C 69/618*** *(2006.01)* ***C08J 5/00*** *(2006.01)*
***C08K 5/134*** *(2006.01)* ***B65D 81/24*** *(2006.01)*

(21) Application number: **10150716.8**

(22) Date of filing: **14.01.2010**

(54) **Packaging body**

Verpackungskörper

Corps d'emballage

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **29.01.2009 JP 2009017861**

(43) Date of publication of application:
**04.08.2010 Bulletin 2010/31**

(73) Proprietor: **Sumitomo Chemical Company, Limited**
**Tokyo 104-8260 (JP)**

(72) Inventor: **Sato, Natsuko**
**Osaka (JP)**

(74) Representative: **Heunemann, Dieter**
**Vossius & Partner**
**Siebertstrasse 4**
**81675 München (DE)**

(56) References cited:
**EP-A2- 0 818 505** **US-A- 4 732 923**

EP 2 213 368 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to a packaging body.

For improving the processing stability of a thermoplastic polymer composition, it is known, for example, to blend a compound such as 2-t-butyl-6-(3-t-butyl-2-hydroxy-5-methylbenzyl)-4-methylph enyl acrylate and the like into a thermoplastic polymer (patent document 1).

(patent document 1) JP-A No. 62-18445

It was desired to develop a technology for further improving the processing stability of a thermoplastic polymer composition.

Under such conditions, the present inventors have investigated and resultantly completed the present invention described below.

That is, the present invention provides the following [1] to [3].

[1] A packaging body comprising a bag body filled with a compound of the following formula (1) and further filled with an inert gas so as to occupy 80 vol% or more with respect to the total capacity of an internal space formed by the bag body:

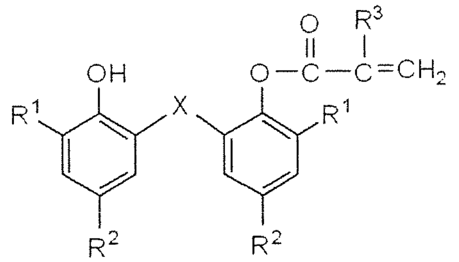

(1)

in the formula (1), $R^1$ and $R^2$ represent each independently an alkyl group having 1 to 8 carbon atoms or cycloalkyl group having 5 to 8 carbon atoms, $R^3$ represents a hydrogen atom or methyl group, and X represents a single bond, sulfur atom, oxygen atom, alkylidene group having 1 to 8 carbon atoms or cycloalkylidene group having 5 to 8 carbon atoms,

wherein the bag body is made of a gas barrier wrapping material and (0007)

wherein the gas barrier wrapping material has an oxygen transmission rate of 200 [$cm^3 \cdot 25\ \mu m \cdot m^{-2} \cdot day^{-1} \cdot atm^{-1}$] or less.

[2] The packaging body according to any one of [1] to [3] wherein the compound of the formula (1) is 2,4-di-t-amyl-6-[1-(3,5-di-t-amyl-2-hydroxyphenyl)ethyl]phe nyl acrylate or 2-t-butyl-6-(3-t-butyl-2-hydroxy-5-methylbenzyl)-4-methylph enyl acrylate.

[3] A method of producing a packaging body, comprising a filling step of filling a bag body with a compound of the following formula (1),

an introduction step of further introducing an inert gas into the bag body so that the gas occupies 80 vol% or more with respect to the total capacity of an internal space formed by the bag body: and

a sealing step of sealing the bag body obtained via the filling step and the introduction step:

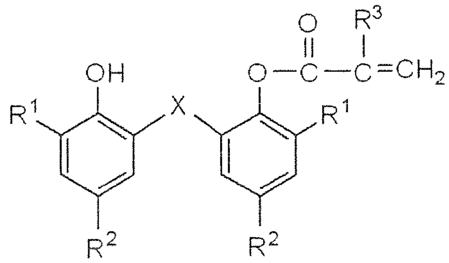

(1)

in the formula (1), $R^1$ and $R^2$ represent each independently an alkyl group having 1 to 8 carbon atoms or cycloalkyl group having 5 to 8 carbon atoms, $R^3$ represents a hydrogen atom or methyl group, and X represents a single bond, sulfur atom, oxygen atom, alkylidene group having 1 to 8 carbon atoms or cycloalkylidene group having 5 to 8 carbon

atoms.

The present invention will be described in detail below.

The compound used in the packaging body of the present invention (hereinafter, described as the present packaging body in some cases) is a compound of the above-described formula (1) (hereinafter, described as compound (1) in some cases).

In the compound (1), $R^1$ and $R^2$ represent each independently an alkyl group having 1 to 8 carbon atoms or cycloalkyl group having 5 to 8 carbon atoms.

Examples of the alkyl group include a methyl group, ethyl group, n-propyl group, i-propyl group, n-butyl group, i-butyl group, t-butyl group, n-pentyl group, i-pentyl group, t-pentyl group, 2-ethylhexyl group and the like, and examples of the cycloalkyl group include a cyclopentyl group, cyclohexyl group, cyclooctyl group, 3-methylcyclopentyl group, 4-methylcyclopentyl group, 3-methylcyclohexyl group and the like, and tertiary alkyl groups such as a t-butyl group and t-pentyl group and the like are preferable and a t-pentyl group is more preferable.

In the formula (1), $R^3$ represents a hydrogen atom or methyl group, and a hydrogen atom is preferable.

In the formula (1), X represents a single bond; a sulfur atom; an oxygen atom; an alkylidene group having 1 to 8 carbon atoms such as a methylene group, ethylidene group, propylidene group, butylidene group and the like; or a cycloalkylidene group having 5 to 8 carbon atoms such as a cyclopentylidene group, cyclohexylidene group .

Examples of the compound (1) include 2-[1-(2-hydroxy-3,5-di-t-pentylphenyl)ethyl]-4,6-di-t-penty lphenyl acrylate, 2-t-butyl-6-(3-t-butyl-2-hydroxy-5-methylbenzyl)-4-methylph enyl acrylate, 2,4-di-t-butyl-6-[1-(3,5-di-t-butyl-2-hydroxyphenyl)ethyl]p henyl acrylate, 2-t-butyl-6-[1-(3-t-butyl-2-hydroxy-5-methylphenyl)ethyl]-4 -methylphenyl acrylate, 2-t-butyl-6-[1-(3-t-butyl-2-hydroxy-5-methylphenyl)propyl]-4-methylphenyl acrylate, 2-t-butyl-6-[1-(3-t-butyl-2-hydroxy-5-propylphenyl)ethyl]-4 -propylphenyl acrylate, 2-t-butyl-6-[1-(3-t-butyl-2-hydroxy-5-isopropylphenyl)ethyl ]-4-isopropylphenyl acrylate and the like, preferably, 2-[1-(2-hydroxy-3,5-di-t-pentylphenyl)ethyl]-4,6-di-t-penty lphenyl acrylate or 2-t-butyl-6-(3-t-butyl-2-hydroxy-5-methylbenzyl)-4-methylph enyl acrylate.

As the gas barrier wrapping material, wrapping materials are mentioned having an oxygen transmission rate of preferably 200 [$cm^3 \cdot 25\ \mu m \cdot m^{-2} \cdot day^{-1} \cdot atm^{-1}$] or less, more preferably 100 [$cm^3 \cdot 25\ \mu m \cdot m^{-2} \cdot day^{-1} \cdot atm^{-1}$] or less, further preferably 50 [$cm^3 \cdot 25\ \mu m \cdot m^{-2} \cdot day^{-1} \cdot atm^{-1}$] or less.

Examples of the material of the wrapping material include plastic films such as a polyvinyl alcohol film, polyamide film, polyacrylonitrile film, polyethylene terephthalate film, nylon film, moisture-proof cellophane film, polyvinylidene chloride film and the like; copolymerized polyolefin copolymer films such as a vinyl chloride-propylene copolymerized plastic film, ethylene-vinyl alcohol copolymerized plastic film and the like; and composite films thereof.

As the material of the gas barrier wrapping material, there can be used laminate films obtained by vapor-depositing a metal such as aluminum onto an oxygen transmissible film such as a polyethylene film, and polyethylene terephthalate so as to give an oxygen transmission rate of 200 [$cm^3 \cdot 25\ \mu m \cdot m^{-2} \cdot day^{-1} \cdot atm^{-1}$] or less, and also laminate films obtained by further vapor-depositing a metal such as aluminum and the like onto a film already having an oxygen transmission rate of 200 [$cm^3 \cdot 25\ \mu m \cdot m^{-2} \cdot day^{-1} \cdot atm^{-1}$] or less.

As the material of the gas barrier wrapping material, laminate films vapor-deposited with aluminum are preferable and laminate films obtained by vapor-deposing a metal such as aluminum onto an oxygen transmissible film are more preferable.

The present packaging body is obtained by filling a bag body with a compound (1).

As the above-described bag body a gas barrier wrapping material may be sealed by thermal compression bond and the like to obtain a bag body.

An inert gas is further filled so as to occupy 80 vol% or more with respect to the total capacity of an internal space formed by the bag body. The capacity occupied by the inert gas is preferably 83 vol% or more, more preferably 90 vol% or more.

Here, the phrase "with respect to the total capacity of an internal space formed by the bag body" means a volume capacity of a transient or permanent space formed insulating from an outer space by the gas barrier wrapping material forming the bag body irrespective of sealing or non-sealing.

Examples of the inert gas include rare gases such as helium, and argon; nitrogen, and carbon dioxide. Preferable is nitrogen or argon, and more preferable is nitrogen.

As the method of producing the present packaging body, for example, a production method comprising a filling step of filling a bag body with a compound (1), an introduction step of further introducing an inert gas into the bag body so that the gas occupies 80 vol% or more with respect to the total capacity of an internal space formed by the bag body, and a sealing step of sealing the bag body obtained via the filling step and the introduction step, and other methods are mentioned.

Other two steps than the sealing step, that is, the filling step and the introduction step may be carried out in any order, and it may be advantageous that the filling step and the introduction step are carried out in any order before conducting the sealing step.

Specifically mentioned are a method in which a filling step of filling a bag body with a compound (1) is carried out, then, an introduction step of introducing an inert gas so as to occupy 80 vol% or more with respect to the total capacity of an internal space formed by the bag body obtained in the filling step is carried out, and the bag body obtained in the introduction step is sealed;
and a method in which an introduction step of introducing an inert gas so as to occupy 80 vol% or more with respect to the total capacity of an internal space formed by the bag body is carried out in a place substituted with an inert gas of 80 vol% or more, a filling step of filling the bag body obtained in the introduction step with a compound (1) is carried out in a place substituted with an inert gas of 80 vol% or more.

Examples of the sealing method to be used in the sealing step include a method of performing thermal compression bond, a method of sealing with a zipper, chuck or the like, a method of suturing with a thread or the like, and other methods.

A thermoplastic polymer composition prepared by removing a compound (1) from the packaging body of the present invention, then, blending this compound (1) into a thermoplastic polymer without leaving for a long period of time is excellent in processing stability and has a coloration suppressing effect.

Such a thermoplastic polymer is not particularly restricted providing it is a commercially available thermoplastic polymer, and thermoplastic polymers obtained by solution polymerization are preferable. Of them, polypropylene resins such as an ethylene-propylene copolymer and the like; polyethylene resins (high density polyethylene(HD-PE), low density polyethylene(LD-PE), linear low density polyethylene(LLDPE)), methylpentene polymer, ethylene-ethyl acrylate copolymer, ethylene-vinyl acetate copolymer, polystyrenes (polystyrene such as poly(p-methylstyrene), poly($\alpha$-methylstyrene) , and acrylonitrile-styrene copolymer, acrylonitrile-butadiene-styrene copolymer, special acryl rubber-acrylonitrile-styrene copolymer, acrylonitrile-chlorinated polyethylene-styrene copolymer, styrene-butadiene copolymer and the like), chlorinated polyethylene, polychloroprene, chlorinated rubber, polyvinyl chloride, polyvinylidene chloride, methacrylic resin, ethylene-vinyl alcohol copolymer, fluorine resin, polyacetal, grafted polyphenylene ether resin, polyphenylene sulfide resin, polyurethane, polyamide, polyester resins (for example, polyethylene terephthalate, polybutylene terephthalate ), polycarbonate, polyacrylate, polysulfone, polyether ether ketone, polyether sulfone, aromatic polyester resin, diallyl phthalate prepolymer, silicone resin, 1,2-polybutadiene, polyisoprene, butadiene/acrylonitrile copolymer, ethylene-methyl methacrylate copolymer and the like are mentioned, and particularly, preferable from the standpoint of good molding processability are polyethylene resins, polypropylene resins and polystyrenes, and especially, polypropylene resins, acrylonitrile-butadiene-styrene copolymer and styrene-butadiene copolymer are more preferable.

The addition amount of the compound (1) is preferably 0.01 to 5 parts by weight, more preferably 0.02 to 2 parts by weight, further preferably 0.05 to 1 part by weight, still further preferably 0.1 to 0.6 parts by weight with respect to 100 parts by weight of a thermoplastic polymer.

The above-described thermoplastic polymer composition is suitable for, for example, food packaging containers, daily use miscellaneous goods, electronic and electric parts and materials, parts and materials of transport machines such as automobile etc.
A thermoplastic polymer composition prepared by removing a compound of the formula (1) preserved in the present packaging body from the packaging body, then, blending this compound (1) into a thermoplastic polymer without leaving for a long period of time is excellent in processing stability and has a coloration suppressing effect.

## EXAMPLES

The present invention will be described in detail by examples and reference examples mentioned below, but the present invention is not limited to them. Parts and % are by weight unless otherwise stated.

### (Example 1)

<Fabrication of packaging body>

In a glove box previously adjusted to nitrogen gas 83 vol% and oxygen gas 17 vol%, 12 g of 2,4-di-t-amyl-6-[1-(3,5-di-t-amyl-2-hydroxyphenyl)ethyl]phe nyl acrylate (Sumilizer GS(F), registered trademark, manufactured by Sumitomo Chemical Co., Ltd.) was filled in a bag body with chuck (115 mm $\times$ 90 mm, Seisannipponsha Ltd., Lamizip AL-9) made of a gas barrier wrapping material (oxygen transmission rate: 0.1 to 1 [$cm^3 \cdot 25\ \mu m \cdot m^{-2} \cdot day^{-1} \cdot atm^{-1}$]) composed of polyethylene terephthalate (outer layer) /aluminum vapor deposited layer/polyethylene (inner layer), then, the chuck was hermetically closed to obtain a packaging body.

<Preservation>

The packaging body obtained above was preserved in a ventilation constant temperature oven at 50°C for 1

month.

<Coloring property test>

The above-described compound was removed from the packaging body after preservation described above, and 0.5 parts of the removed compound and 100 parts of a propylene-ethylene block copolymer (MI (230°C, load: 2.16 kg): 3 g/10 min., manufactured by Sumitomo Chemical Co., Ltd.) were dry-blended, then, the resultant blend was kneaded using a 30 mmφ single screw extruder (Tanabe Plastics Machinery Co. , Ltd., VS30-28 type extruder) under conditions of 230°C and a screw revolution of 50 rpm, to obtain pellets of a thermoplastic polymer composition.

The resultant pellets were subjected to measurement of Yellowness Index (YI) using a colorimeter (CM-3500d, manufactured by Konica Minolta Holdings, Inc.) according to JIS K7105, to obtain a value of -6.07. Smaller the YI value, weaker the coloration.

<Processing stability>

The pellets obtained in <Coloring property test> were subjected to measurement of melt flow rate (MFR, 280°C, load: 2.16 kg) using Melt Indexer (L217-E14011, manufactured by TechnoSeven Co. , Ltd.), to obtain a value of 9.91. Smaller the MFR value, more excellent the processing stability.

(Examples 2 to 4, Reference Example 1)

Thermoplastic polymer compositions were produced according to the same method as in Example 1 excepting that the content of a nitrogen gas in the glove box was changed as described in Table 1, and the performances of the resultant thermoplastic polymer compositions were evaluated. These results are shown in Table 1 together with those of Example 1.

For "improvement rate", the above-described compound allowed to stand still on a petri dish without being filled in a bag body was preserved in a ventilation constant temperature oven at 50°C for 1 month and the resultant reference substance was subjected to the coloring property test and the processing stability test according to the same procedure as in Example 1 (hereinafter, described as Reference Example 1 in some cases), and the improvement rate was calculated based on the obtained results. In Example 1, the improvement rate of the coloring property test was:

$$\{-6.01-(-5.39)\}/(-5.39)\times 100 = 12\ (\%)$$

since YI in Reference Example 1 was -5.39 and the improvement rate of the processing stability was:

$$(10.82-10.08)/10.82\times 100 = 6.8\ (\%)$$

since MFR in Reference Example 1 was 10.82.

(Table 1)

| | nitrogen content (vol%)*1 | YI | YI improvement rate(%) | MFR | MFR improvement rate(%) |
|---|---|---|---|---|---|
| Example 1 | 83 | -6.07 | 13 | 9.91 | 8.4 |
| Example 2 | 90 | -6.09 | 13 | 9.80 | 9.4 |
| Example 3 | 96 | -6.26 | 16 | 9.79 | 9.5 |
| Example 4 | 100 | -6.50 | 21 | 9.73 | 10.1 |
| Reference Example 1 | 79 | -5.39 | 0 | 10.82 | 0 |
| *1: Capacity ratio of an inert gas occupying with respect to the total capacity of an internal space formed by the bag body | | | | | |

(Example 5)

Under an air atmosphere, 12 g of 2,4-di-t-amyl-6-[1-(3,5-di-t-amyl-2-hydroxyphenyl)ethyl]phe nyl acrylate (Sumilizer GS(F), registered trademark, manufactured by Sumitomo Chemical Co., Ltd.) was filled in a bag body with chuck (115 mm × 90 mm, Seisannipponsha Ltd., Lamizip AL-9) made of a gas barrier wrapping material (oxygen transmission rate: 0.1 to 1 [$cm^3 \cdot 25\ \mu m \cdot m^{-2} \cdot day^{-1} \cdot atm^{-1}$]) composed of polyethylene terephthalate (outer layer)/aluminum vapor deposited layer/polyethylene (inner layer), then, a nitrogen gas was introduced into the resultant bag body for about 1 minute from a nitrogen cylinder of which pressure had been controlled to 1 $kg/cm^2$ (9.8 Pa), then, the chuck was hermetically closed to obtain a packaging body.

A thermoplastic polymer composition prepared by removing a compound of the formula (1) preserved in the present packaging body from the packaging body, then, blending this compound (1) into a thermoplastic polymer without leaving for a long period of time is excellent in processing stability and has a coloration suppressing effect.

## Claims

1. A packaging body comprising a bag body filled with a compound of the following formula (1) and further filled with an inert gas so as to occupy 80 vol% or more with respect to the total capacity of an internal space formed by the bag body, wherein the bag body is made of a gas barrier wrapping material which has an oxygen transmission rate of 200 [$cm^3 \cdot 25\ \mu m \cdot m^{-2} \cdot day^{-1} \cdot atm^{-1}$] or less:

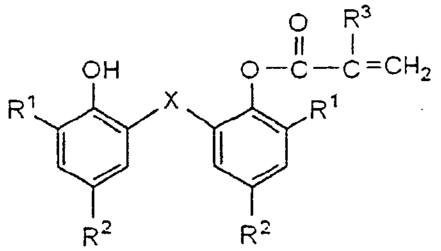

(1)

(in the formula (1), $R^1$ and $R^2$ represent each independently an alkyl group having 1 to 8 carbon atoms or cycloalkyl group having 5 to 8 carbon atoms, $R^3$ represents a hydrogen atom or methyl group, and X represents a single bond, sulfur atom, oxygen atom, alkylidene group having 1 to 8 carbon atoms or cycloalkylidene group having 5 to 8 carbon atoms.

2. The packaging body according to Claim 1 wherein the compound of the formula (1) is 2,4-di-t-amyl-6-[1-(3,5-di-t-amyl-2-hydroxyphenyl)ethyl]phe nyl acrylate or 2-t-butyl-6-(3-t-butyl-2-hydroxy-5-methylbenzyl)-4-methylph enyl acrylate.

3. A method of producing a packaging body, comprising
a filling step of filling a bag body with a compound of the following formula (1),
an introduction step of further introducing an inert gas into the bag body so that the gas occupies 80 vol% or more with respect to the total capacity of an internal space formed by the bag body: and
a sealing step of sealing the bag body obtained via the filling step and the introduction step wherein the bag body is made of a gas barrier wrapping material which has an oxygen transmission rate of 200 [$cm^3 \cdot 25\ \mu m \cdot m^{-2} \cdot day^{-1} \cdot atm^{-1}$] or less:

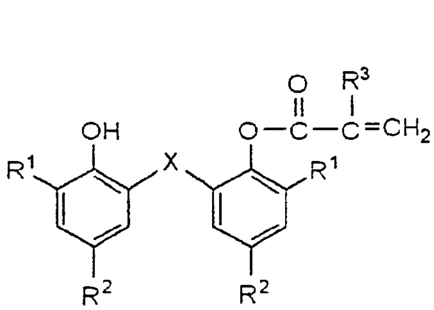

(1)

in the formula (1), $R^1$ and $R^2$ represent each independently an alkyl group having 1 to 8 carbon atoms or cycloalkyl group having 5 to 8 carbon atoms, $R^3$ represents a hydrogen atom or methyl group, and X represents a single bond, sulfur atom, oxygen atom, alkylidene group having 1 to 8 carbon atoms or cycloalkylidene group having 5 to 8 carbon atoms.

**Patentansprüche**

1. Ein Verpackungskörper umfassend einen Behältniskörper, der mit einer Verbindung der folgenden Formel (1) gefüllt ist und der ferner mit einem Inertgas gefüllt ist, so dass 80 Vol.-% oder mehr in Bezug zu der Gesamtkapazität eines Innenraums, der durch den Behältniskörper gebildet wird, ausgefüllt sind, wobei der Behältniskörper aus einem Gas-Barriere-Verpackungsmaterial hergestellt ist, welches eine Sauerstoffdurchlässiglceitsrate von 200 [$cm^3 \cdot 25\ \mu m \cdot m^{-2} \cdot Tag^{-1} \cdot atm^{-1}$] oder weniger aufweist:

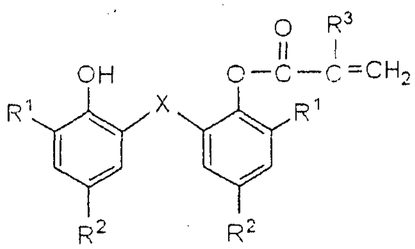

(1)

wobei in der der Formel (1), $R^1$ und $R^2$ jeweils unabhängig einen Alkylrest mit 1 bis 8 Kohlenstoffatomen oder einen Cycloalkylrest mit 5 bis 8 Kohlenstoffatomen darstellen, $R^3$ ein Wasserstoffatom oder eine Methylgruppe darstellt und X eine Einfachbindung, ein Schwefelatom, ein Sauerstoffatom, einen Alkylidenrest mit 1 bis 8 Kohlenstoffatomen oder einen Cycloalkylidenrest mit 5 bis 8 Kohlenstoffatomen darstellt.

2. Der Verpackungskörper gemäß Anspruch 1, wobei die Verbindung der Formel (1) 2,4-Di-t-amyl-6-[1-(3,5-di-t-amyl-2-hydroxyphenyl)ethyl]phenylacrylat oder 2-t-Butyl-6-(3-t-butyl-2-hydroxy-5-methylbenzyl)-4-methylphenylacrylat ist.

3. Ein Verfahren zur Herstellung eines Verpackungskörpers, umfassend einen Befüllschritt des Füllens eines Behältniskörpers mit einer Verbindung der folgenden Formel (1), einen Einführungsschritt des ferner Einführens eines Inertgases in den Behältnisskörper, so dass das Gas 80 Vol.-% oder mehr in Bezug zu der Gesamtkapazität eines Innenraums, der durch den Behältniskörper gebildet wird, einnimmt, und
einen Verschlussschritt des Verschließens des Behältniskörpers, der durch den Befüllschritt und den Einführungsschritt erhalten werde, wobei der Behältnisskörper aus einem Gas-Barriere-Verpackungsmaterial hergestellt ist, welches eine Sauerstoffdurchlässigkeitsrate von 200 [$cm^3 \cdot 25\ \mu m \cdot m^{-2} \cdot Tag^{-1} \cdot atm^{-1}$] oder weniger aufweist:

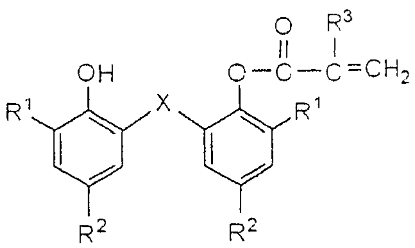

(1)

wobei in der Formel (1), $R^1$ und $R^2$ jeweils unabhängig einen Alkylrest mit 1 bis 8 Kohlenstoffatomen oder einen Cycloalkylrest mit 5 bis 8 Kohlenstoffatomen darstellen, $R^3$ ein Wasserstoffatom oder eine Methylgruppe darstellt und X eine Einfachbindung, ein Schwefelatom, ein Sauerstoffatom, einen Alkylidenrest mit 1 bis 8 Kohlenstoffatomen oder einen Cycloalkylidenrest mit 5 bis 8 Kohlenstoffatomen darstellt.

## Revendications

1. Corps d'emballage comprenant un corps en forme de sac rempli avec un composé répondant à la formule (1) indiquée ci-après et rempli ultérieurement avec un gaz inerte de façon à occuper 80 % en volume ou plus par rapport à la capacité totale de l'espace interne formé par le corps en forme de sac, le corps en forme de sac étant réalisé en un matériau d'emballage procurant une barrière contre les gaz, dont le taux de transmission à l'oxygène s'élève à 200 [$cm^3 \cdot 25\ \mu m \cdot m^{-2} \cdot jour^{-1} \cdot atm^{-1}$] ou moins :

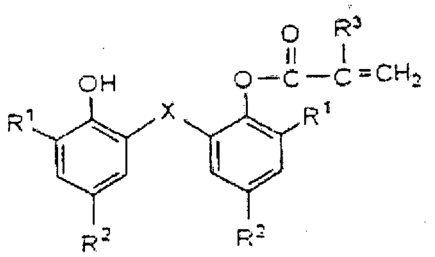

(1)

formule (I) dans laquelle $R^1$ et $R^2$ représentent, chacun indépendamment l'un de l'autre, un groupe alkyle contenant de 1 à 8 atomes de carbone ou un groupe cycloalkyle contenant de 5 à 8 atomes de carbone, $R^3$ représente un atome d'hydrogène ou un groupe méthyle, et X représente une liaison simple, un atome de soufre, un atome d'oxygène, un groupe alkylidène contenant de 1 à 8 atomes de carbone ou un groupe cycloalkylidène contenant de 5 à 8 atomes de carbone.

2. Corps d'emballage selon la revendication 1, dans lequel le composé répondant à la formule (I) est l'acrylate de 2,4-di-tert.-amyl-6-[1-(3,5-di-tert.-amyl-2-hydroxyphényl)éthyl]phényle ou l'acrylate de 2-tert.-butyl-6-(3-tert.-butyl-2-hydroxy-5-méthylbenzyl)-4-méthylphényle.

3. Procédé de fabrication d'un corps d'emballage, comprenant
une étape de remplissage consistant à remplir un corps en forme de sac avec un composé répondant à la formule (1) indiquée ci-après ;
une étape d'introduction consistant à introduire ultérieurement un gaz inerte dans le corps en forme de sac de telle sorte que le gaz occupe 80 % en volume ou plus de la capacité totale de l'espace interne formé par le corps en forme de sac ; et
une étape d'étanchéisation consistant à rendre étanche le corps en forme de sac que l'on obtient via l'étape de remplissage et via l'étape d'introduction, le corps en forme de sac étant réalisé en un matériau d'emballage procurant une barrière contre les gaz, dont le taux de transmission à l'oxygène s'élève à 200 [$cm^3 \cdot 25\ \mu m \cdot m^{-2} \cdot jour^{-1} \cdot atm^{-1}$] ou moins :

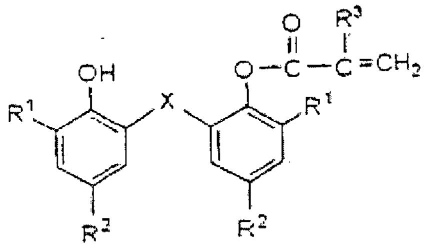

(1)

formule (I) dans laquelle $R^1$ et $R^2$ représentent, chacun indépendamment l'un de l'autre, un groupe alkyle contenant de 1 à 8 atomes de carbone ou un groupe cycloalkyle contenant de 5 à 8 atomes de carbone, $R^3$ représente un atome d'hydrogène ou un groupe méthyle, et X représente une liaison simple, un atome de soufre, un atome d'oxygène, un groupe alkylidène contenant de 1 à 8 atomes de carbone ou un groupe cycloalkylidène contenant de 5 à 8 atomes de carbone.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 62018445 A